(19) Europäisches Patentamt
European Patent Office
Office européen des brevets

(11) EP 1 502 580 B1

# (12) FASCICULE DE BREVET EUROPEEN

(45) Date de publication et mention de la délivrance du brevet:
**23.01.2008 Bulletin 2008/04**

(51) Int Cl.:
***A61K 8/35*** (2006.01) ***A61Q 5/10*** (2006.01)

(21) Numéro de dépôt: **04291872.2**

(22) Date de dépôt: **23.07.2004**

(54) **Utilisation en coloration de triones vicinales aromatiques polycycliques à cycles condensés**

Verwendung in der Färbung von polycyclischen aromatischen vicinalen Trionen mit kondensierten Ringen

Use in dyeing of aromatic polycyclic vicinal triones with condensed cycles

(84) Etats contractants désignés:
**AT BE BG CH CY CZ DE DK EE ES FI FR GB GR HU IE IT LI LU MC NL PL PT RO SE SI SK TR**

(30) Priorité: **25.07.2003 FR 0309172**
**04.03.2004 FR 0402246**

(43) Date de publication de la demande:
**02.02.2005 Bulletin 2005/05**

(73) Titulaire: **L'ORÉAL**
**75008 Paris (FR)**

(72) Inventeurs:
- **Plos, Grégory**
  **Tokyo 158-0097 (JP)**
- **Gourlaouen, Luc**
  **92600 Asnières (FR)**

(74) Mandataire: **Dossmann, Gérard**
**Bureau Casalonga & Josse**
**Bayerstrasse 71/73**
**80335 München (DE)**

(56) Documents cités:
**WO-A-01/97764** **DE-A- 4 335 627**

- **DATABASE CAPLUS [Online] XP002284669 extrait de STN Database accession no. 1989: 596731**
- **DATABASE CAPLUS [Online] XP002284670 extrait de STN Database accession no. 1981: 14638**
- **DATABASE CAPLUS [Online] XP002284671 extrait de STN Database accession no. 1980: 514956**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 502 580 B1

## Description

La présente invention concerne des compositions de coloration des matières kératiniques, en particulier des compositions de coloration capillaire contenant au moins une trione vicinale aromatique polycyclique à cycles condensés, associée, de préférence, à un composé à fonction amine primaire ou secondaire ou à un composé à fonction méthylène activée, un procédé de coloration utilisant de telles compositions et un agent de coloration multi-composants servant à la mise en oeuvre d'un tel procédé.

Depuis longtemps, de nombreuses personnes cherchent à modifier la couleur de leur peau, de leurs cils ou de leurs cheveux et en particulier à masquer leurs cheveux blancs. Pour ce faire, plusieurs technologies ont été développées.

Il est connu de teindre les fibres kératiniques humaines, et en particulier les cheveux, avec des compositions tinctoriales contenant des précurseurs de colorants d'oxydation, appelés généralement bases d'oxydation. Ces bases d'oxydation sont des composés incolores ou faiblement colorés qui, associés à des produits oxydants, donnent naissance par un processus de condensation oxydative à des composés colorés. Ces colorants sont insolubles et sont piégés à l'intérieur de la fibre capillaire.

On sait également que l'on peut faire varier les nuances obtenues avec ces bases d'oxydation en les associant à des coupleurs ou modificateurs de coloration. La variété des molécules mises en jeu au niveau des bases d'oxydation et des coupleurs permet l'obtention d'une riche palette de couleurs.

Les colorations obtenues présentent une bonne ténacité aux shampooings. Cependant, la réaction d'oxydation se fait à l'aide de produits oxydants tels l'eau oxygénée en milieu basique. Ces agents oxydants attaquent la kératine des cheveux dont les propriétés cosmétiques et mécaniques peuvent se dégrader fortement en cas de colorations répétées.

Il est aussi connu de teindre les fibres kératiniques humaines par une coloration directe consistant à appliquer sur les fibres kératiniques des colorants directs qui sont des molécules colorées et colorantes ayant une affinité pour les fibres. On peut citer à titre d'exemples de colorants directs utilisés classiquement les colorants nitrés, benzéniques, anthraquinoniques, nitropyridiniques, azoïques, azoïques cationiques, xanthéniques, acridiniques, aziniques ou de type triarylméthane ou des colorants naturels.

Les colorations obtenues ainsi sont, certes, très chromatiques et n'entraînent pas une dégradation chimique de la kératine, mais présentent l'inconvénient de n'être que temporaires ou semi-permanentes, c'est-à-dire de s'estomper au mieux après seulement 4 à 5 shampooings.

Il subsiste par conséquent un besoin de systèmes et de procédés de coloration qui permettent l'obtention de bonnes ténacités sans impliquer l'utilisation d'agents oxydants susceptibles de dégrader les matières kératiniques.

La demanderesse a découvert avec surprise que l'utilisation de certaines triones vicinales aromatiques polycycliques, décrites plus en détail ci-dessous permettait de colorer les matières kératiniques, et en particulier les cheveux, avec des ténacités équivalentes voire supérieures à celles obtenues par coloration d'oxydation et ceci en l'absence d'agents oxydants forts préservant ainsi parfaitement les matières kératiniques.

Les triones vicinales aromatiques polycycliques mentionnées ci-dessus sont utilisées de préférence en combinaison avec des composés à hydrogène labile, tels que des amines primaires ou secondaires ou des composés à fonction méthylène activée.

Les colorations obtenues ainsi présentent de bonnes chromaticités et se distinguent en particulier par une excellente ténacité au lavage (plusieurs dizaines de shampooings).

L'invention a donc pour objet l'utilisation, pour la coloration de matières kératiniques, d'une composition contenant, dans un milieu approprié pour la teinture, au moins un composé de formule (I) ou (II) ou sa forme tautomère :

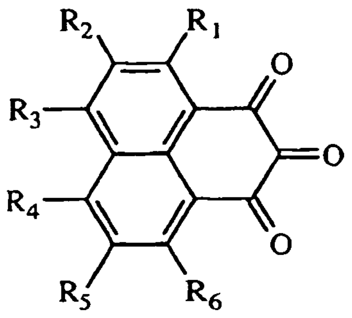

(I)

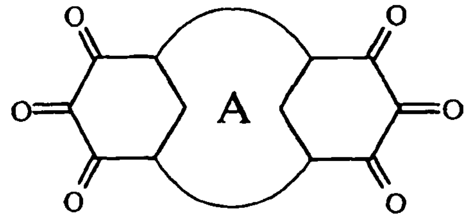

(II)

dans lesquelles

$R^1$, $R^2$, $R^3$, $R^4$, $R^5$ et $R^6$ représentent, indépendamment les uns des autres, chacun un atome d'hydrogène, un atome d'halogène, un groupe alkyle en $C_{1-6}$, hydroxy, alcoxy en $C_{1-6}$, benzoxy éventuellement substitué, aryloxy éventuellement substitué, amino, mono- ou di-(alkyle en $C_{1-6}$)-amino, mono- ou di-(hydroxyalkyle en $C_{1-6}$)-amino, thio, alkylthio en $C_{1-6}$, thioalkyle en $C_{1-6}$, (alkyle en $C_{1-6}$)-carbonyle, hydrogénocarbonyle, hydroxycarbonyle, (alcoxy en $C_{1-6}$)-carbonyle, nitro, sulfonato et tri(alkyle en $C_{1-6}$)-ammonio, ou un groupe aromatique comportant au moins 5 chaînons, monocyclique ou polycyclique à cycles condensés ou non condensés, comportant éventuellement un ou plusieurs hétéroatomes choisis parmi N, O, S et P, et portant éventuellement un ou plusieurs substituants choisis parmi les atomes d'halogène, les groupes alkyle en $C_{1-6}$, hydroxy, alcoxy en $C_{1-6}$, amino, mono- ou di-(alkyle en $C_{1-6}$)-amino, mono- ou di-(hydroxyalkyle en $C_{1-6}$)-amino, thio, alkylthio en $C_{1-6}$, thioalkyle en $C_{1-6}$, (alkyle en $C_{1-6}$)-carbonyle, hydrogénocarbonyle, hydroxycarbonyle, (alcoxy en $C_{1-6}$)-carbonyle, nitro, sulfonato, tri(alkyle en $C_{1-6}$)-ammonio, phénoxy, imidazolyle et pyridinyle, ou $R^1$ et $R^2$, $R^2$ et $R^3$, $R^3$ et $R^4$, $R_4$ et $R^5$, ou $R^5$ et $R^6$ forment ensemble, deux par deux, un groupe $-O-CH_2-O-$ ou un cycle à 5 ou 6 chaînons, aromatique ou non aromatique, contenant éventuellement un ou plusieurs hétéroatomes choisis parmi N, O, S et P, et portant éventuellement un ou plusieurs substituants choisis parmi les atomes d'halogène, les groupes alkyle en $C_{1-6}$, hydroxy, alcoxy en $C_{1-6}$, amino, mono- ou di-(alkyle en $C_{1-6}$)-amino, mono- ou di-(hydroxyalkyle en $C_{1-6}$)-amino, thio, alkylthio en $C_{1-6}$, thioalkyle en $C_{1-6}$, (alkyle en $C_{1-6}$)-carbonyle, hydrogénocarbonyle, hydroxycarbonyle, (alcoxy en $C_{1-6}$)-carbonyle, nitro, sulfonato, tri (alkyle en $C_{1-6}$)-ammonio, imidazolyle et pyridinyle, et

A représente un système polycyclique aromatique, comportant éventuellement un ou plusieurs hétéroatomes choisis parmi N, O, S et P et portant éventuellement un ou plusieurs substituants choisis parmi les substituants des groupes aromatiques représentés par $R^1$ à $R^6$ ci-dessus.

De telles compositions sont en particulier utiles pour la teinture des fibres kératiniques, notamment les cheveux.

Dans un mode de réalisation préféré de la présente invention, A est choisi de manière à former par condensation avec les deux cycles en $C_6$-trione un système à électrons $\pi$ délocalisés.

Les atomes d'halogène englobent les atomes de chlore, de brome, de fluor et d'iode.

Les composés de formule (I) et (II) englobent, bien entendu, également les sels d'addition d'acides et les sels d'addition de bases correspondants.

Les triones vicinales aromatiques polycyliques de formule (I) et (II) ci-dessus sont utilisées dans la présente invention dans un milieu cosmétiquement acceptable contenant généralement une fraction importante d'eau. Lorsqu'elles sont dissoutes dans un tel milieu aqueux, ces composés sont en équilibre d'hydratation avec la forme diol géminé (ou hydrate de carbonyle) correspondant aux formules (Ibis) et (IIbis) suivantes :

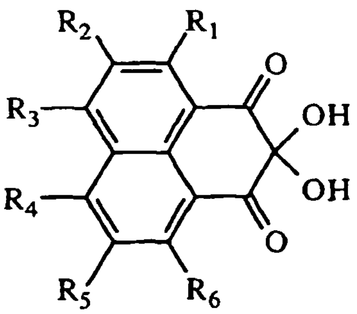

(Ibis)

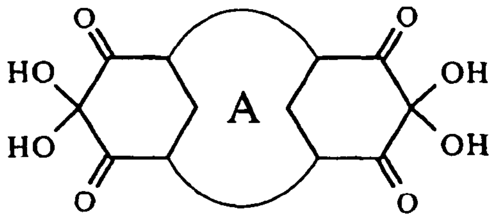

(IIbis)

Lorsqu'il est question, dans la présente demande, de triones de formule (I) et (II), celles-ci englobent par conséquent toujours non seulement les composés de formule (I) et (II) mais également les formes hydratées correspondantes de formule (Ibis) et (IIbis).

Dans un mode de réalisation préféré des composés de formule (II) de la présente invention, A représente un noyau naphtalène formant par condensation avec les deux cycles en $C_6$ adjacents une pyrène-1,2,3,6,7,8-hexone de formule (III) :

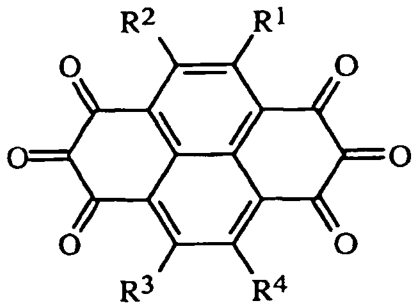

(III)

où les substituants $R^1$ à $R^4$ ont les définitions indiquées ci-dessus pour $R^1$ à $R^6$.

Dans un autre mode de réalisation préféré des composés de formule (II) de la présente invention, A représente un noyau pérylène formant, par condensation avec les deux cycles en $C_6$ adjacents, une dibenzo-[cd,lm]pérylène-1,2,3,8,9,10-hexone de formule (IV)

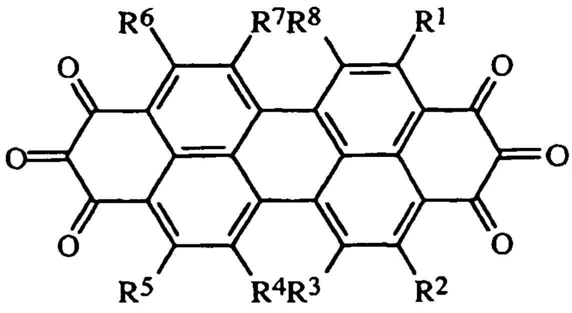

(IV)

dans laquelle les substituants $R^1$ à $R^8$ ont la signification indiquée ci-dessus pour les substituants $R^1$ à $R^6$ de la formule (I).

Des exemples préférés de triones vicinales aromatiques polycycliques à cycles condensés, utilisables conformément à la présente invention pour la coloration des fibres capillaires sont les suivants :

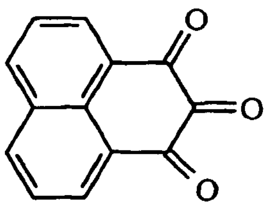

(a)

1H-phénalène-1,2,3-trione

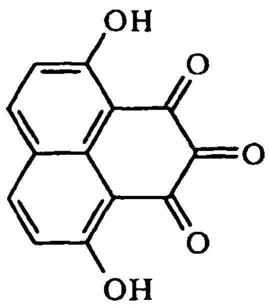

(b)

4,9-dihydroxy-1H-phénalène-1,2,3-trione

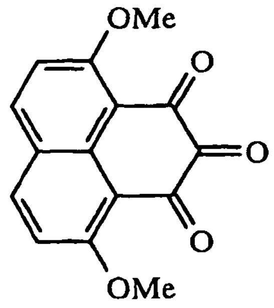

(c)

4,9-diméthoxy-1H-phénalène-1,2,3-trione

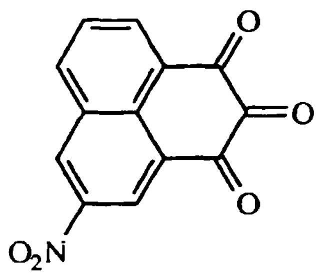

(d)

5-nitro-1H-phénalène-1,2,3-trione

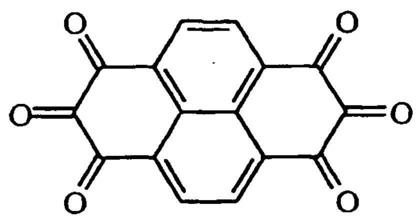

(e)

1,2,3,6,7,8-pyrènehexone

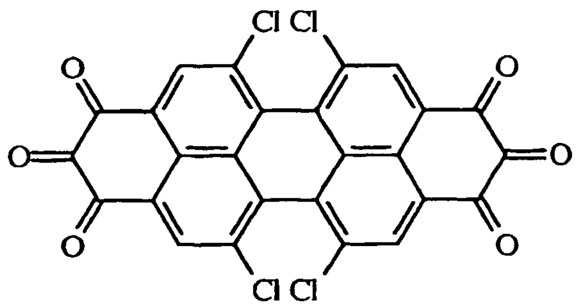

(f)

5,6,12,13-tétrachloro-dibenzo[cd,lm]pérylène-1,2,3,8,9,10-hexone

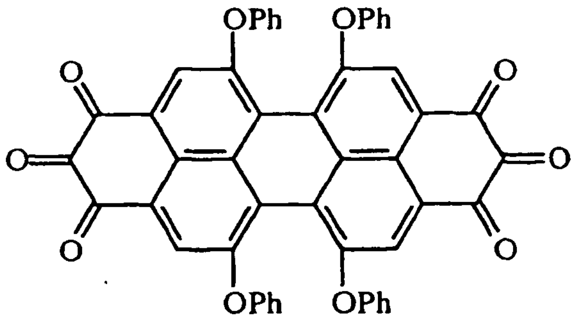

(g)

5,6,12,13-tétraphénoxy-dibenzo[cd,lm]pérylène-1,2,3,8,9,10-hexone

Les triones vicinales aromatiques polycycliques de formule (I) et (II) utilisées dans la présente invention sont connues. La synthèse des composés exemplifiés (a) à (g) est décrite par exemple dans les deux publications suivantes :

- 1H-phénalène-1,2,3-trione : G. Errera Gazz. Chim. Ital. 431, 583 (1913)
- 1,2,3,6,7,8-pyrènehexone : R. Gleiter, R. Kraemer, H. Irngartinger, C. Bissinger, J. Org. Chem. 57, 252 (1992).

Conformément à la présente invention, les triones vicinales aromatiques polycycliques de formule (I) et (II) décrites ci-dessus peuvent être utilisées seules pour la coloration des matières kératiniques. En effet, ces composés sont capables de générer des molécules colorées avec les fonctions amine de la kératine (réaction colorée).

On peut aussi utiliser conjointement des composés de formule (I) et/ou (II) avec au moins un activateur qui permet de modifier la cinétique de réaction du composé ninhydrine avec la matière kératinique. Un tel activateur peut être un agent oxydant, un agent réducteur, des acides de brönsted, un catalyseur métallique tel que les catalyseurs à base de métal de transition tel que le fer, le platine, le palladium, des protéines notamment des enzymes, des composés modifiant la force ionique du milieu tels que des sels de NaCl, des composés à hydrogène labile choisi parmi ceux comportant une fonction amine primaire ou secondaire et ceux comportant une fonction méthylène activée. On peut, bien entendu, utiliser également un mélange de tels composés.

Les composés à fonction amine primaire ou amine secondaire sont de préférence des amines aromatiques.

On peut citer à titre d'exemples de telles amines aromatiques la N,N-diméthyl-p-phénylènediamine, la N,N-diéthyl-p-phénylènediamine, la N-(2-hydroxy-éthyl)-N-éthyl-p-phénylènediamine, la N,N-bis-(2-hydroxyéthyl)-p-phénylène-diamine, la N-(2-méthoxyéthyl)-p-phénylènediamine, la 2,3-, 2,4- ou 2,5-dichloro-p-phénylène-diamine, la 2-chloro-p-phénylènediamine, le dibromhydrate de 2,5-dihydroxy-4-morpholinoaniline, le 2-, 3- ou 4-aminophénol, le 2-aminométhyl-4-aminophénol, le 2-hydroxyméthyl-4-aminophénol, l'ortho-phénylènediamine, la p-phénylènediamine, l'ortho-toluènediamine, le 2,5-diaminotoluène, le 2,5-diaminophénol, le 2,5-diaminophénéthol, le 4-amino-3-méthylphénol, le 2-(2,5-diaminophényl)-éthanol, le 2,4-diaminophénoxyéthanol, le 2-(2,5-diaminophénoxy)-éthanol, la 4-méthylaminoaniline, la 3-amino-4-(2'-hydroxyéthyloxy)aniline, la 3,4-méthylènediaminoaniline, la 3,4-méthylènedioxyaniline, le 3-amino-2,4-dichlorophénol, le 4-méthylaminophénol, le 2-méthyl-5-aminophénol, le 3-méthyl-4-aminophénol, le 2-méthyl-5-(2-hydroxyéthylamino)phénol, le 6-méthyl-3-amino-2-chlorophénol, le 2-méthyl-5-amino-4-chlorophénol, le 3,4-méthylènedioxyphénol, le 5-(2-hydroxyéthylamino)-4-méthoxy-2-méthylphénol, le 4-amino-2-hydroxyméthylphénol, le 1,3-diamino-2,4-diméthoxybenzène, l'acide 2-, 3-, 4-aminobenzoïque, l'acide 2-amino-, 3-amino ou 4-aminophénylacétique, l'acide 2,3-, 2,4-, 2,5-, 3,4- ou 3,5-diaminobenzoïque, l'acide 4-amino- ou 5-amino-salicylique, l'acide 3-amino-4-hydroxybenzoïque, l'acide 4-amino-3-hydroxybenzoïque, l'acide 2-amino-, 3-amino ou 4-aminobenzènesulfonique, l'acide 3-amino-4-hydroxybenzènesulfonique, l'acide 4-amino-3-hydroxynaphtalène-1-sulfonique, l'acide 6-amino-7-hydroxynaphtalène-2-sulfonique, l'acide 7-amino-4-hydroxynaphtalène-2-sulfonique, l'acide 4-amino-5-hydroxynaphtalène-2,7-disulfonique, l'acide 3-amino-2-naphtoïque, l'acide 3-aminophtalique, l'acide 5-aminoisophtalique, le 1,3,5-triaminobenzène, le 1,2,4-triaminobenzène, le 1,2,4,5-tétraaminobenzène, le 2,4,5-triaminophénol, le pentaaminobenzène, l'hexaaminobenzène, le 2,4,6-triaminorésorcinol, le 4,5-diaminopyrochatécol, le 4,6-diaminopyrogallol, le 3,5-diamino-4-hydroxypyrochatécol, et les anilines aromatiques et phénols aromatiques comportant un autre résidu aromatique, correspondant à la formule (II)

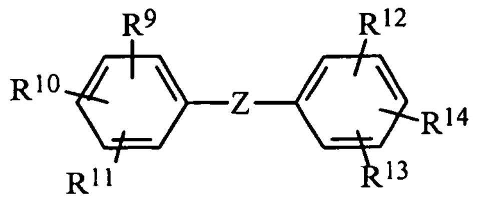

(II)

dans laquelle

- $R^9$ représente un groupe hydroxy ou amino éventuellement substitués par un groupe alkyle en $C_{1-4}$, hydroxyalkyle en $C_{1-4}$ ou (alcoxy en $C_{1-4}$)-(alkyle en $C_{1-4}$),
- $R^{10}$, $R^{11}$, $R^{12}$, $R^{13}$ et $R^{14}$ représentent chacun indépendamment un atome d'hydrogène, un groupe hydroxy ou un groupe amino, éventuellement substitués par un groupe alkyle en $C_{1-4}$, hydroxyalkyle en $C_{1-4}$ ou (alcoxy en $C_{1-4}$)-(alkyle en $C_{1-4}$), ou un groupe acide carboxylique ou sulfonique,
- Z représente une liaison directe, une chaîne hydrocarbonée en $C_{1-4}$, saturée ou insaturée, éventuellement hydroxylée, un groupe carbonyle, sulfonyle ou imino, un atome d'oxygène ou de soufre, ou un groupe de formule $Q-(CH_2-P-CH_2-Q')_o$ où P représente une liaison directe ou un groupe $-CH_2-$ ou -CHOH-, Q et Q' représentent, indépendamment l'un de l'autre, un atome d'oxygène, un groupe $NR^{15}$ où $R^{15}$ représente un atome d'hydrogène, un groupe alkyle en $C_{1-4}$ ou hydroxyalkyle en $C_{1-4}$, ou un groupe $O-(CH_2)_pNH$ ou $NH-(CH_2)_{p'}-O$ où p et p' valent 2 ou 3 et o est un nombre entre 1 et 4.

Les amines primaires ou secondaires non-aromatiques sont par exemple le 2-aminoéthanol, la 2-méthoxyéthylamine, la 2-éthoxyéthylamine, le 2-(2-aminoéthoxy)-éthanol, le 2- ou 3-aminopropanol, la 2,3-dihydroxypropylamine, la 4-hydroxypropylamine, le 2-aminopropane-1,3-diol, le 2-amino-2-méthylpropanol, le 2-amino-2-méthylpropane-1,3-diol, le 2-amino-2-hydroxyméthylpropane-1,3-diol, la tétrahydropentylamine, les pentahydroxyhexylamines telles que la glucamine, la D-glucosamine, la D-galactosamine, le 1,2-diaminoéthane, le 1,2- ou 1,3-diaminopropane, le 1,3-diamino-2-propanol, la 2-(2-aminoéthylamino)-éthylamine, le 2-(2-aminoéthylamino)éthanol, la 3-(2-aminoéthylamino)-propylamine et le 3-(2-aminoéthylamino)propanol.

Les composés à fonction méthylène activée sont choisis par exemple parmi les suivants : l'iodure de 1,2,3,3-tétraméthyl-3H-indolium, le p-toluènesulfonate de 1,2,3,3-tétraméthyl-3H-indolium, le méthanesulfonate de 1,2,3,3-tétraméthyl-3H-indolium, la 1,3,3-triméthyl-2-méthylène-indoline, l'iodure de 2,3-diméthylbenzothiazolium, le p-toluènesulfonate de 2,3-diméthyl-benzothiazolium, la rhodanine, l'acide rhodanine-3-acétique, l'iodure de 1-éthyl-2-quinaldinium, l'iodure de 1-méthyl-2-quinaldinium, l'acide barbiturique, l'acide thiobarbiturique, l'acide 1,3-diméthylthiobarbiturique, l'acide diéthylthiobarbiturique, l'oxindole, l'acétate de 3-indoxyle, la coumarone et la 1-méthyl-3-phényl-2-pyrazolinone.

Ces amines primaires et secondaires et ces composés à fonctions méthylène activée ainsi que d'autres composés à hydrogène labile sont décrits également dans les demandes de brevet DE 43 17 855, DE 197 17 222, DE 198 45 481 et DE 197 45 355 où ils sont utilisés pour la coloration des fibres kératiniques en combinaison avec des dérivés de ninhydrine.

Lorsque les triones vicinales aromatiques polycycliques de formule (I) et (II) sont utilisées en combinaison avec une amine primaire ou secondaire ou avec un composé à fonction méthylène activée, il est nécessaire de conserver ces différents réactifs séparément pour éviter une réaction colorée prématurée. La mise en contact des réactifs ne se fait alors qu'immédiatement avant application sur les cheveux, par mélange extemporané de deux compositions contenant respectivement les triones vicinales de formule (I) et (II) et les composés à hydrogène labile.

L'invention a par conséquent également pour objet un agent de coloration multi-composants comportant

- en tant que premier composant, une composition (a) contenant au moins une trione vicinale aromatique polycyclique de formule (I) ou (II), et
- en tant que deuxième composant, une composition (b) contenant au moins un composé à fonction amine primaire ou secondaire ou au moins un composé à fonction méthylène activée, tels que décrits ci-dessus.

Cet agent de coloration multi-composants se présente de préférence sous forme d'un kit multi-compartiments, avec au moins un premier compartiment contenant le premier composant (composition (a)) et au moins un deuxième compartiment contenant le deuxième composant (composition (b)).

L'invention a également pour objet une composition cosmétique tinctoriale contenant au moins une trione vicinale aromatique polycyclique de formule (I) ou (II) et au moins un principe actif cosmétique.

Les principes actifs cosmétiques présents dans les compositions cosmétiques de la présente invention sont choisis par exemple parmi les vitamines, les saccharides, les oligosaccharides, les polysaccharides hydrolysés ou non, modifiés ou non, les acides aminés, les oligopeptides, les peptides, les protéines hydrolysées ou non, modifiées ou non, les polyacides aminés, les enzymes, les acides et alcools gras ramifiés ou non, les cires animales, végétales ou minérales, les céramides et les pseudo-céramides, les acides organiques hydroxylés, les filtres UV, les agents antioxydants et les agents anti-radicaux libres, les agents chélatants, les agents antipelliculaires, les agents régulateurs de séborrhée, les agents apaisants, les agents tensioactifs cationiques, anioniques, non-ioniques ou amphotères, les polymères cationiques, anioniques, neutres ou amphotères, les silicones organomodifiées ou non, les huiles minérales, végétales ou animales, les polyisobutènes et poly($\alpha$-oléfines), les esters gras, les polymères anioniques sous forme dissoute ou dispersée, les polymères non-ioniques sous forme dissoute ou dispersés, les agents réducteurs, les solvants, les colorants capillaires tels que les colorants directs ou les précurseurs de coloration d'oxydation (bases et/ou coupleurs) différents des composés à fonction amine primaire ou secondaire revendiqués, les agents oxydants tels que le peroxyde d'hydrogène éventuellement associé à des persels, les pigments et leurs mélanges.

Le principe actif cosmétique est présent de préférence à raison de 0,001 à 50 % en poids, en particulier de 0,01 à 20 % en poids, et idéalement à raison de 0,1 à 10 % en poids, rapporté au poids total de la composition cosmétique.

Dans un mode de réalisation préféré de la composition cosmétique tinctoriale selon l'invention, le principe actif cosmétique est un agent tensioactif et/ou un agent polymérique (polymère), ces agents pouvant être de nature non-ionique, cationique, anionique ou amphotère.

Il ressort de ce qui précède que les compositions de coloration capillaire utilisées dans la présente invention sont stables au stockage lorsqu'elles contiennent, comme seuls réactifs de coloration, des triones vicinales aromatiques polycycliques de formule (I) ou (II), mais qu'elles doivent être préparées immédiatement avant emploi, lorsqu'elles contiennent à la fois des triones vicinales aromatiques polycycliques de formule (I) ou (II) et des composés à hydrogène labile tels que des amines primaires et secondaires ou des composés à fonction méthylène activée.

Ces compositions de coloration prêtes à l'emploi, qu'elles soient stables au stockage ou préparées immédiatement avant emploi, ont de préférence un pH compris entre 2 et 12, et en particulier entre 3 et 11.

Leur teneur en triones vicinales aromatiques polycycliques de formule (I) ou (II) est de préférence comprise entre 0,0001 % et 30 % en poids.

Les composés à hydrogène labile utilisés en combinaison avec les triones de formule (I) ou (II) sont de préférence présents à raison de 0,0001 % à 30 % rapporté au poids total de la composition.

La présente invention a en outre pour objet un procédé de coloration capillaire comprenant l'application, sur les cheveux, d'une composition de coloration capillaire prête à l'emploi telle que décrite ci-dessus. Cette composition est laissée en contact avec les fibres capillaires pendant un temps suffisant pour l'obtention de la coloration désirée. Ce temps de pose est généralement compris entre 5 minutes et 1 heure, et en particulier entre 15 et 30 minutes. La réaction colorée entre les triones vicinales aromatiques polycycliques et les fonctions amine de la kératine ou les composés à hydrogène labile éventuellement présents, peut être accélérée par chauffage des cheveux imprégnées avec la composition de coloration. La température de chauffage ne dépasse de préférence pas 80 °C, et est en particulier inférieure ou égale à 60 °C.

Après obtention de la coloration souhaitée, les cheveux sont rincés et lavés.

Lorsqu'on utilise des composés à hydrogène labile tels que des amines primaires ou secondaires ou des composés à fonction méthylène activée, l'application des réactifs participant à la réaction colorée peut également se faire en deux temps, autrement dit on peut appliquer successivement deux compositions différentes contenant respectivement au moins une trione vicinale aromatique polycyclique de formule (I) ou (II) et au moins un composé comportant une fonction amine primaire ou secondaire ou une fonction méthylène activée.

La présente invention a par conséquent également pour objet un procédé de coloration en deux temps comprenant le fait d'appliquer sur les cheveux l'une après l'autre, dans n'importe quel ordre, une composition (a) et une composition (b) telles que définies ci-dessus pour l'agent de coloration multi-composants.

Cette application séparée des deux compositions réactives présente l'avantage d'éviter la manipulation de compositions colorées et diminue ainsi les risques de souillure de matériaux tels que les vêtements.

La demanderesse a constaté que l'on obtenait également des colorations capillaires satisfaisantes lorsqu'une étape de rinçage intermédiaire était insérée entre l'application de la première composition et l'application de la deuxième composition.

De manière analogue à celle décrite ci-dessus, les cheveux imprégnés de composition (a) et/ou (b) peuvent être chauffés, de préférence jusqu'à une température de 80 °C, et en particulier jusqu'à une température ne dépassant pas 60 °C, un tel chauffage permettant d'accélérer la réaction colorée et de raccourcir le temps de pose.

**Exemple**

On prépare la composition suivante :

| | |
|---|---|
| 1-H-phénatène-1,2,3-trione (composé de formule (a)) | $10^{-2}$ moles |
| Ethanol | 50 g |
| NaOH | q.s.p pH 7 |
| Eau distillée | q.s.p. 100 g |

On applique cette composition sur deux mèches de cheveux naturels et permanentés à 90 % de cheveux blancs, de 1 g chacune. Le rapport de bain est de 5, le temps de pose de 30 minutes et la température de 60 °C. A la fin du temps de pose, les mèches sont rincées, puis lavées avec un shampooing standard.

L'intensité de coloration est évaluée par colorimétrie selon le système CIELAB à l'aide d'un colorimètre CM3600d de Minolta (illuminant D65, angle d'observation : 10 °, composante spéculaire incluse).

Le système de notation CIELAB définit un espace colorimétrique dans lequel chaque couleur est définie par 3 paramètres (L*, a* et b*) :

- le paramètre L* reflète la clarté de la couleur, la valeur de L* étant égale à 0 pour le noir et égale à 1 pour le blanc absolu. Plus la valeur de L* est élevée, moins la coloration est intense.
- le paramètre a* correspond à l'axe du couple antagoniste vert-rouge et le paramètre b* à l'axe du couple antagoniste bleu-jaune.

Le tableau ci-dessous montre les paramètres L*, a* et b* des mèches de cheveux naturels et de cheveux permanentés avant et après la montée de la couleur, ainsi que ΔE défini par l'équation ci-après :

$$\Delta E = \sqrt{(L^*_{final} - L^*_{initial})^2 + (a^*_{final} - a^*_{initial})^2 + (b^*_{final} - b^*_{initial})^2}$$

ΔE reflète la variation globale de la couleur. Sa valeur est d'autant plus grande que la variation de couleur est importante.

| Cheveux | | L* | a* | b* | ΔE | Couleur |
|---|---|---|---|---|---|---|
| naturels | Avant coloration | 60,84 | 0,03 | 11,63 | - | - |
| naturels | Après coloration | 25,71 | -2,45 | 6,54 | 35,58 | mat |
| permanentés | Avant coloration | 61,78 | 0,33 | 12,74 | - | - |
| permanentés | Après coloration | 18,93 | -0,14 | 2,33 | 44,11 | mat |

## Revendications

1. Utilisation pour la coloration de matières kératiniques d'une composition contenant, dans un milieu approprié pour la teinture, au moins un composé de formule (I) ou (II) :

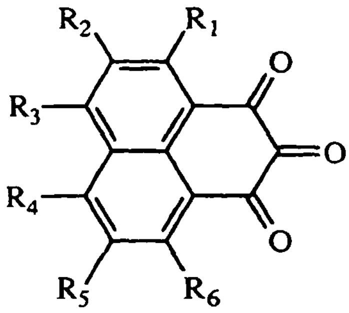 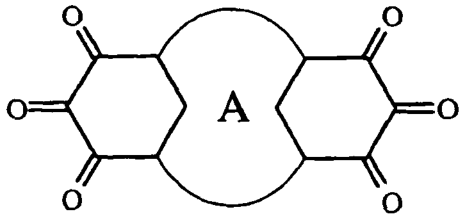

(I) (II)

dans lesquelles

$R^1$, $R^2$, $R^3$, $R^4$, $R^5$ et $R^6$ représentent, indépendamment les uns des autres, chacun un atome d'hydrogène, un atome d'halogène, un groupe alkyle en $C_{1-6}$, hydroxy, alcoxy en $C_{1-6}$, benzoxy éventuellement substitué, aryloxy éventuellement substitué, amino, mono- ou di-(alkyle en $C_{1-6}$)-amino, mono- ou di-(hydroxyalkyle en $C_{1-6}$)-amino, thio, alkylthio en $C_{1-6}$, thioalkyle en $C_{1-6}$, (alkyle en $C_{1-6}$)-carbonyle, hydrogénocarbonyle, hydroxycarbonyle, (alcoxy en $C_{1-6}$)-carbonyle, nitro, sulfonato et tri(alkyle en $C_{1-6}$)-ammonio, ou un groupe aromatique comportant au moins 5 chaînons, monocyclique ou polycyclique à cycles condensés ou non condensés, comportant éventuellement un ou plusieurs hétéroatomes choisis parmi N, O, S et P, et portant éventuellement un ou plusieurs substituants choisis parmi les atomes d'halogène, les groupes alkyle en $C_{1-6}$, hydroxy, alcoxy en $C_{1-6}$, amino, mono- ou di-(alkyle en $C_{1-6}$)-amino, mono- ou di-(hydroxyalkyle en $C_{1-6}$)-amino, thio, alkylthio en $C_{1-6}$, thioalkyle en $C_{1-6}$, (alkyle en C $_{1-6}$)-carbonyle, hydrogénocarbonyle, hydroxycarbonyle, (alcoxy en $C_{1-6}$)-carbonyle, nitro, sulfonato, tri(alkyle en $C_{1-6}$)-ammonio, phénoxy, imidazolyle et pyridinyle, ou $R^1$ et $R^2$, $R^2$ et $R^3$, $R^3$ et W, $R_4$ et $R^5$, ou $R^5$ et $R^6$ forment ensemble, deux par deux, un groupe $-O-CH_2-O-$ ou un cycle à 5 ou 6 chaînons, aromatique ou non aromatique, contenant éventuellement un ou plusieurs hétéroatomes choisis parmi N, O, S et P, et portant éventuellement un ou plusieurs substituants choisis parmi les atomes d'halogène, les groupes alkyle en $C_{1-6}$, hydroxy, alcoxy en $C_{1-6}$, amino, mono- ou di-(alkyle en $C_{1-6}$)-amino, mono- ou di-(hydroxyalkyle en $C_{1-6}$)-amino, thio, alkylthio en $C_{1-6}$, thioalkyle en $C_{1-6}$, (alkyle en $C_{1-6}$)-carbonyle, hydrogénocarbonyle, hydroxycarbonyle, (alcoxy en $C_{1-6}$)-carbonyle, nitro, sulfonato, tri(alkyle en $C_{1-6}$)-ammonio, imidazolyle et pyridinyle, et

A représente un système polycyclique aromatique comportant éventuellement un ou plusieurs hétéroatomes choisis parmi N, O, S et P et portant éventuellement un ou plusieurs substituants choisis parmi les substituants des groupes aromatiques représentés par $R^1$ à $R^6$ ci-dessus.

**2.** Utilisation selon la revendication 1, **caractérisée par le fait que** A représente un noyau naphtalène qui forme, par condensation avec les deux cycles en $C_6$ adjacent, une pyrène-1,2,3,6,7,8-hexone de formule (III)

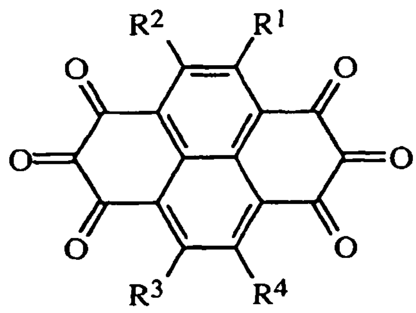

(III)

dans laquelle les substituants $R^1$ à $R^4$ ont la signification indiquée dans la revendication 1.

3. Utilisation selon la revendication 1, **caractérisée par le fait que** A représente un noyau pérylène formant, par condensation avec les deux cycles en $C_6$ adjacents, une dibenzo-[cd,lm]pérylène-1,2,3,8,9,10-hexone de formule (IV) :

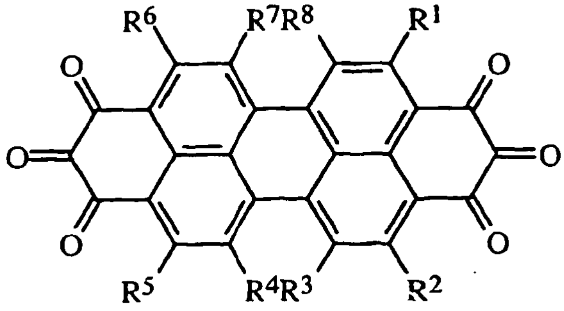

(IV)

dans laquelle les substituants $R^1$ à $R^8$ ont la signification indiquée dans la revendication 1 pour les substituants $R^1$ à $R^6$ de la formule (I).

4. Utilisation selon l'une des revendications 1 à 3, **caractérisée par le fait que** la composition comprend en outre au moins un activateur qui permet de modifier la cinétique de réaction du composé ninhydrine avec la matière kératinique.

5. Utilisation selon la revendication 4, **caractérisée par le fait que** l'activateur est choisi parmi un agent oxydant, un agent réducteur, des acides de brönsted, un catalyseur métallique, des protéines, des composés modifiant la force ionique du milieu, des composés à hydrogène labile choisi parmi ceux comportant une fonction amine primaire ou secondaire et ceux comportant une fonction méthylène activée et un mélange de ceux-ci.

6. Utilisation selon la revendication 5, **caractérisée par le fait que** le composé comportant une fonction amine primaire ou secondaire est une amine aromatique choisie parmi la N,N-diméthyl-p-phénylènediamine, la N,N-diéthyl-p-phénylènediamine, la N-(2-hydroxy-éthyl)-N-éthyl-p-phénylènediamine, la N,N-bis-(2-hydroxyéthyl)-p-phénylène-diamine, la N-(2-méthoxyéthyl)-p-phénylènediamine, la 2,3-, 2,4- ou 2,5-dichloro-p-phénylène-diamine, la 2-chloro-p-phénylènediamine, le dibromhydrate de 2,5-dihydroxy-4-morpholinoaniline, le 2-, 3- ou 4-aminophénol, le 2-aminométhyl-4-aminophénol, le 2-hydroxyméthyl-4-aminophénol, l'ortho-phénylènediamine, la p-phénylènediamine, l'ortho-toluènediamine, le 2,5-diaminotoluène, le 2,5-diaminophénol, le 2,5-diaminophénéthol, le 4-amino-3-méthylphénol, le 2-(2,5-diaminophényl)-éthanol, le 2,4-diaminophénoxyéthanol, le 2-(2,5-diaminophénoxy)-éthanol, la 4-méthylaminoaniline, la 3-amino-4-(2'-hydroxyéthyloxy)aniline, la 3,4-méthylènediaminoaniline, la 3,4-méthylènedioxyaniline, le 3-amino-2,4-dichlorophénol, le 4-méthylaminophénol, le 2-méthyl-5-aminophénol, le 3-méthyl-4-aminophénol, le 2-méthyl-5-(2-hydroxyéthylamino)phénol, le 6-méthyl-3-amino-2-chlorophénol, le 2-méthyl-5-amino-4-chlorophénol, le 3,4-méthylènedioxyphénol, le 5-(2-hydroxyéthylamino)-4-méthoxy-2-méthylphénol, le 4-amino-2-hydroxyméthylphénol, le 1,3-diamino-2,4-diméthoxybenzène, l'acide 2-, 3-, 4-aminobenzoïque, l'acide 2-amino-, 3-amino ou 4-aminophénylacétique, l'acide 2,3-, 2,4-, 2,5-, 3,4- ou 3,5-diaminobenzoïque, l'acide 4-amino- ou 5-amino-salicylique, l'acide 3-amino-4-hydroxybenzoïque, l'acide 4-amino-3-hydroxybenzoïque, l'acide 2-amino-, 3-amino ou 4-aminobenzènesulfonique, l'acide 3-amino-4-hydroxybenzènesulfonique, l'acide 4-amino-3-hydroxynaphtalène-1-sulfonique, l'acide 6-amino-7-hydroxynaphtalène-2-sulfonique, l'acide 7-amino-4-hydroxynaphtalène-2-sulfonique, l'acide 4-amino-5-hydroxynaphtalène-2,7-disulfonique, l'acide 3-amino-2-naphtoïque, l'acide 3-aminophtalique, l'acide 5-aminoisophtalique, le 1,3,5-triaminobenzène, le 1,2,4-triaminobenzène, le 1,2,4,5-tétraaminobenzène, le 2,4,5-triaminophénol, le pentaaminobenzène, l'hexaaminobenzène, le 2,4,6-triaminorésorcinol, le 4,5-diaminopyrochatécol, le 4,6-diaminopyrogallol, le 3,5-diamino-4-hydroxypyrochatécol, et les anilines aromatiques et phénols aromatiques comportant un autre résidu aromatique, correspondant à la formule (II)

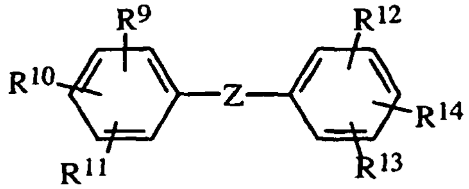

(II)

dans laquelle

$R^9$ représente un groupe hydroxy ou amino éventuellement substitués par un groupe alkyle en $C_{1-4}$, hydroxyalkyle en $C_{1-4}$ ou (alcoxy en $C_{1-4}$)-(alkyle en $C_{1-4}$),
$R^{10}$, $R^{11}$, $R^{12}$, $R^{13}$ et $R^{14}$ représentent chacun indépendamment un atome d'hydrogène, un groupe hydroxy ou un groupe amino, éventuellement substitués par un groupe alkyle en $C_{1-4}$, hydroxyalkyle en $C_{1-4}$ ou (alcoxy en $C_{1-4}$)-(alkyle en $C_{1-4}$), ou un groupe acide carboxylique ou sulfonique,
Z représente une liaison directe, une chaîne hydrocarbonée en $C_{1-4}$, saturée ou insaturée, éventuellement hydroxylée, un groupe carbonyle, sulfonyle ou imino, un atome d'oxygène ou de soufre, ou un groupe de formule $Q-(CH_2-P-CH_2-Q')_o$ où P représente une liaison directe ou un groupe $-CH_2-$ ou -CHOH-, Q et Q' représentent, indépendamment l'un de l'autre, un atome d'oxygène, un groupe $NR^{15}$ où $R^{15}$ représente un atome d'hydrogène, un groupe alkyle en $C_{1-4}$ ou hydroxyalkyle en $C_{1-4}$. ou un groupe $O-(CH_2)_pNH$ ou $NH-(CH_2)_{p'}-O$ où p et p' valent 2 ou 3 et o est un nombre entre 1 et 4

ou une amine aliphatique choisie parmi le 2-aminoéthanol, la 2-méthoxyéthylamine, la 2-éthoxyéthylamine, le 2-(2-aminoéthoxy)-éthanol, le 2- ou 3-aminopropanol, la 2,3-dihydroxypropylamine, la 4-hydroxypropylamine, le 2-aminopropane-1,3-diol, le 2-amino-2-méthylpropanol, le 2-amino-2-méthylpropane-1,3-diol, le 2-amino-2-hydroxyméthylpropane-1,3-diol, la tétrahydropentylamine, les pentahydroxyhexylamines telles que la glucamine, la D-glucosamine, la D-galactosamine, le 1,2-diaminoéthane, le 1,2- ou 1,3-diaminopropane, le 1,3-diamino-2-propanol, la 2-(2-aminoéthylamino)-éthylamine, le 2-(2-aminoéthylamino)éthanol, la 3-(2-aminoéthylamino)-propylamine et le 3-(2-aminoéthylamino)propanol.

**7.** Utilisation selon la revendications 5, **caractérisé par le fait que** le composé à fonction méthylène activée est choisi parmi l'iodure de 1,2,3,3-tétraméthyl-3H-indolium, le p-toluènesulfonate de 1,2,3,3-tétraméthyl-3H-indolium, le méthanesulfonate de 1,2,3,3-tétraméthyl-3H-indolium, la 1,3,3-triméthyl-2-méthylène-indoline, l'iodure de 2,3-diméthylbenzothiazolium, le p-toluènesulfonate de 2,3-diméthyl-benzothiazolium, la rhodanine, l'acide rhodanine-acétique, l'iodure de 1-éthyl-2-quinaldinium, l'iodure de 1-méthyl-2-quinaldinium, l'acide barbiturique, l'acide thiobarbiturique, l'acide 1,3-diméthylthiobarbiturique, l'acide diéthylthiobarbiturique, l'oxindole, l'acétate de 3-indoxyle, la coumarone et la 1-méthyl-3-phényl-2-pyrazinone.

**8.** Utilisation selon l'une quelconque des revendications précédentes, **caractérisée par le fait que** la composition a un pH compris entre 2 et 12, de préférence entre 3 et 11.

**9.** Utilisation selon l'une quelconque des revendications précédentes, **caractérisée par le fait que** la concentration du ou des triones vicinales aromatiques polycycliques de formule (I) ou (II) est comprise entre 0,0001 % et 30 %, rapporté au poids total de la composition.

**10.** Composition cosmétique tinctoriale contenant au moins une trione vicinale aromatique polycyclique de formule (I) ou (II) telle que définie dans la revendication 1, et au moins un agent tensioactif et/ou un agent polymérique de nature non-ionique, cationique, anionique ou amphotère.

**11.** Composition cosmétique tinctoriale prête-à-l'emploi contenant au moins une trione vicinale aromatique polycyclique de formule (I) ou (II) telle que définie dans la revendication 1 et au moins un composé comportant une fonction amine primaire ou secondaire ou au moins un composé à fonction méthylène activée ou un mélange de ceux-ci.

**12.** Agent de coloration multi-composants comportant

- en tant que premier composant, une composition (a) contenant au moins une trione vicinale aromatique polycyclique de formule (I) ou (II) telle que définie dans la revendication 1, et
- en tant que deuxième composant, une composition (b) contenant au moins un activateur tel que défini dans

l'une quelconque des revendications 4 à 7.

**13.** Agent de coloration selon la revendication 12, **caractérisé par le fait qu'**il se présente sous forme d'un kit multi-compartiments, avec au moins un premier compartiment contenant la composition (a) et au moins un deuxième compartiment contenant la composition (b).

**14.** Procédé de coloration capillaire comprenant l'application, sur les cheveux, d'une composition de coloration capillaire selon l'une des revendications 10 et 11, un temps de pose suffisant pour permettre l'obtention de la coloration souhaitée, puis le rinçage et le lavage des cheveux.

**15.** Procédé de coloration capillaire selon la revendication 14, **caractérisé par le fait qu'**il comprend le chauffage des cheveux imprégnés de composition de coloration capillaire jusqu'à une température de 80 °C, de préférence de 60 °C.

**16.** Procédé de coloration capillaire comprenant le fait d'appliquer sur les cheveux l'une après l'autre, dans n'importe quel ordre, une composition (a) et une composition (b) telles que définies dans la revendication 12.

**17.** Procédé de coloration capillaire selon la revendication 16, **caractérisé par le fait qu'**une étape de rinçage intermédiaire est insérée entre l'application de la première composition et l'application de la deuxième composition.

**18.** Procédé de coloration capillaire selon l'une quelconque des revendications 16 ou 17, comprenant le chauffage des cheveux imprégnés avec la composition (a) et/ou (b) jusqu'à une température de 80 °C, de préférence de 60 °C.

**Claims**

**1.** Use, for dyeing keratin materials, of a composition containing, in a medium appropriate for dyeing, at least one compound of formula (I) or (II):

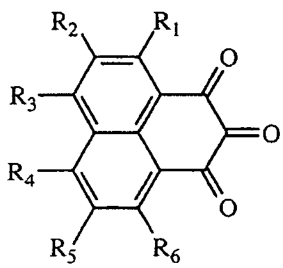

(I)

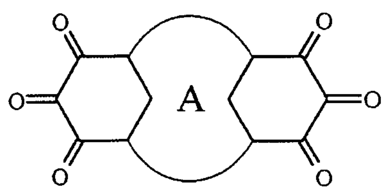

(II)

in which

$R^1$, $R^2$, $R^3$, $R^4$ $R^5$ and $R^6$ each represent, independently of each other, a hydrogen atom, a halogen atom, a $C_{1-6}$ alkyl group, a hydroxyl group, a $C_{1-6}$ alkoxy group, an optionally substituted benzoxy group, an optionally substituted aryloxy group, an amino group, a mono- or di ($C_{1-6}$ alkyl)amino group, a mono- or di($C_{1-6}$ hydroxyalkyl) amino group, a thio group, a $C_{1-6}$ alkylthio group, a $C_{1-6}$ thioalkyl group, a ($C_{1-6}$ alkyl)carbonyl group, a hydrogenocarbonyl group, a hydroxycarbonyl group, a ($C_{1-6}$ alkoxy)carbonyl group, a nitro group, a sulphonato group and a tri($C_{1-6}$ alkyl)ammonio group, or an aromatic group comprising at least 5 members, which is monocyclic or polycyclic, containing fused or non-fused rings, optionally comprising one or more heteroatoms chosen from N, 0, S and P, and optionally bearing one or more substituents chosen from halogen atoms, $C_{1-6}$ alkyl, hydroxyl, $C_{1-6}$ alkoxy, amino, mono- or di($C_{1-6}$ alkyl)amino, mono- or di ($C_{1-6}$ hydroxyalkyl)amino, thio, $C_{1-6}$ alkylthio, $C_{1-6}$ thioalkyl, ($C_{1-6}$ alkyl)carbonyl, hydrogenocarbonyl, hydroxycarbonyl, ($C_{1-6}$ alkoxy)carbonyl, nitro, sulphonato, tri($C_{1-6}$ alkyl)ammonio, phenoxy, imidazolyl and pyridinyl groups, or $R^1$ and $R^2$, $R^2$ and $R^3$, $R^3$ and $R^4$, $R^4$ and $R^5$, or $R^5$ and $R^6$ form together, in pairs, a group $-O-CH_2-O-$ or an aromatic or nonaromatic, 5- or 6-membered ring, optionally containing one or more heteroatoms chosen from N, O, S and P, and optionally bearing one or more substituents chosen from halogen atoms, $C_{1-6}$ alkyl, hydroxyl, $C_{1-6}$ alkoxy, amino, mono- or di($C_{1-6}$ alkyl)

amino, mono- or di($C_{1-6}$ hydroxyalkyl)amino, thio, $C_{1-6}$ alkylthio, $C_{1-6}$ thioalkyl, ($C_{1-6}$ alkyl)carbonyl, hydrogenocarbonyl, hydroxycarbonyl, ($C_{1-6}$ alkoxy)carbonyl, nitro, sulphonato, tri($C_{1-6}$ alkyl)ammonio, imidazolyl and pyridinyl groups, and

A represents an aromatic polycyclic system optionally comprising one or more heteroatoms chosen from N, 0, S and P and optionally bearing one or more substituents chosen from the substituents of the aromatic groups represented by $R^1$ to $R^6$ above.

2. Use according to Claim 1, **characterized in that** A represents a naphthalene ring which forms, by condensation with the two adjacent $C_6$ rings, a pyrene-1,2,3,6,7,8-hexone of formula (III)

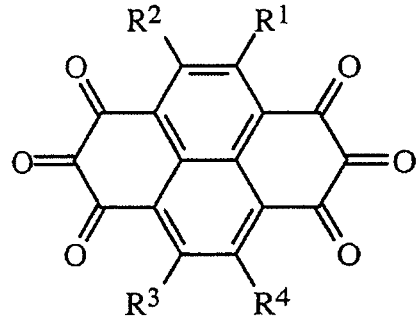

(III)

in which the substituents $R^1$ to $R^4$ have the meaning indicated in Claim 1.

3. Use according to Claim 1, **characterized in that** A represents a perylene ring which forms, by condensation with the two adjacent $C_6$ rings, a dibenzo[cd,lm]perylene-1,2,3,8,9,10-hexone of formula (IV):

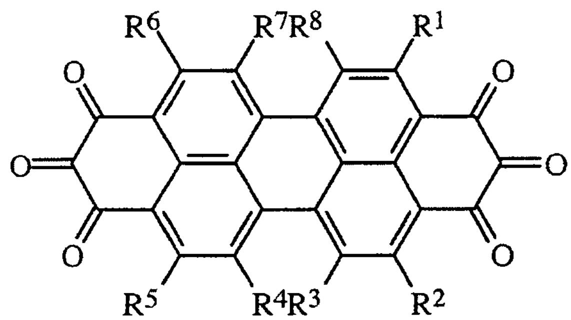

(IV)

in which the substituents $R^1$ to $R^8$ have the meaning indicated in Claim 1 for the substituents $R^1$ to $R^6$ of formula (I).

4. Use according to one of Claims 1 to 3, **characterized in that** the composition additionally comprises at least one activator which makes it possible to modify the kinetics of reaction of the ninhydrin compound with the keratinous material.

5. Use according to Claim 4, **characterized in that** the activator is chosen from an oxidizing agent, a reducing agent, Brönsted acids, a metal catalyst, proteins, compounds which modify the ionic strength of the medium, compounds containing a labile hydrogen chosen from those comprising a primary or secondary amine functional group and those comprising an activated methylene functional group and a mixture thereof.

**6.** Use according to Claim 5, **characterized in that** the compound comprising a primary or secondary amine functional group is an aromatic amine chosen from N,N-dimethyl-p-phenylenediamine, N,N-diethyl-p-phenylenediamine, N-(2-hydroxyethyl)-N-ethyl-p-phenylenediamine, N,N-bis-(2-hydroxyethyl)-p-phenylenediamine, N-(2-methoxyethyl)-p-phenylenediamine, 2,3-, 2,4- or 2,5-dichloro-p-phenylenediamine, 2-chloro-p-phenylenediamine, dibromohydrate of 2,5-dihydroxy-4-morpholinoaniline, 2-, 3- or 4-aminophenol, 2-aminomethyl-4-aminophenol, 2-hydroxymethyl-4-aminophenol, ortho-phenylenediamine, p-phenylenediamine, ortho-toluenediamine, 2,5-diaminotoluene, 2,5-diaminophenol, 2,5-diaminophenethol, 4-amino-3-methylphenol, 2-(2,5-diaminophenyl)ethanol, 2,4-diaminophenoxyethanol, 2-(2,5-diaminophenoxy)ethanol, 4-methylaminoaniline, 3-amino-4-(2'-hydroxyethyloxy)aniline, 3,4-methylenediaminoaniline, 3,4-methylenedioxyaniline, 3-amino-2,4-dichlorophenol, 4-methylaminophenol, 2-methyl-5-aminophenol, 3-methyl-4-aminophenol, 2-methyl-5-(2-hydroxyethylamino)-phenol, 6-methyl-3-amino-2-chlorophenol, 2-methyl-5-amino-4-chlorophenol, 3,4-methylenedioxyphenol, 5-(2-hydroxyethylamino)-4-methoxy-2-methylphenol, 4-amino-2-hydroxymethylphenol, 1,3-diamino-2,4-dimethoxybenzene, 2-, 3-, 4-aminobenzoic acid, 2-amino-, 3-amino- or 4-aminophenylacetic acid, 2,3-, 2,4-, 2,5-, 3,4- or 3,5-diaminobenzoic acid, 4-amino- or 5-aminosalicylic acid, 3-amino-4-hydroxybenzoic acid, 4-amino-3-hydroxybenzoic acid, 2-amino-, 3-amino or 4-aminobenzenesulphonic acid, 3-amino-4-hydroxybenzenesulphonic acid, 4-amino-3-hydroxynaphthalene-1-sulphonic acid, 6-amino-7-hydroxynaphthalene-2-sulphonic acid, 7-amino-4-hydroxynaphthalene-2-sulphonic acid, 4-amino-5-hydroxynaphthalene-2,7-disulphonic acid, 3-amino-2-naphthoic acid, 3-aminophthalic acid, 5-aminoisophthalic acid, 1,3,5-triaminobenzene, 1,2,4-triaminobenzene, 1,2,4,5-tetraaminobenzene, 2,4,5-triaminophenol, pentaaminobenzene, hexaaminobenzene, 2,4,6-triaminoresorcinol, 4,5-diaminopyrocatechol, 4,6-diaminopyrogallol, 3,5-diamino-4-hydroxypyrocatechol, and aromatic anilines and aromatic phenols comprising another aromatic residue, corresponding to formula (II)

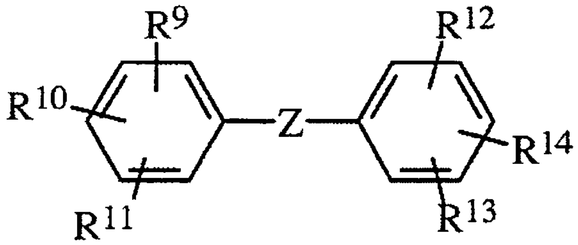

(II)

in which

$R^9$ represents a hydroxyl or amino group optionally substituted with a $C_{1-4}$ alkyl, $C_{1-4}$ hydroxyalkyl or ($C_{1-4}$ alkoxy)-($C_{1-4}$ alkyl) group,
$R^{10}$, $R^{11}$, $R^{12}$, $R^{13}$ and $R^{14}$ each independently represent a hydrogen atom, a hydroxyl group or an amino group, optionally substituted with a $C_{1-4}$ alkyl, $C_{1-4}$ hydroxyalkyl or ($C_{1-4}$ alkoxy)-($C_{1-4}$ alkyl) group, or a carboxylic or sulphonic acid group,
Z represents a direct bond, a $C_{1-4}$ hydrocarbon chain which is saturated or unsaturated, optionally hydroxylated, a carbonyl, sulphonyl or imino group, an oxygen or sulphur atom, or a group of formula $Q-(CH_2-P-CH_2-Q')_o$ where P represents a direct bond or a group $-CH_2-$ or $-CHOH-$, Q and Q' represent, independently of each other, an oxygen atom, a group $NR^{15}$ where $R^{15}$ represents a hydrogen atom, a $C_{1-4}$ alkyl or $C_{1-4}$ hydroxyalkyl group or a group $O-(CH_2)_pNH$ or $NH-(CH_2)_{p'}-O$ where p and p' are 2 or 3 and o is a number between 1 and 4 or an aliphatic amine chosen from 2-aminoethanol, 2-methoxyethylamine, 2-ethoxyethylamine, 2-(2-aminoethoxy)ethanol, 2- or 3-aminopropanol, 2,3-dihydroxypropylamine, 4-hydroxypropylamine, 2-aminopropane-1,3-diol, 2-amino-2-methylpropanol, 2-amino-2-methylpropane-1,3-diol, 2-amino-2-hydroxymethylpropane-1,3-diol, tetrahydropentylamine, pentahydroxyhexylamines such as glucamine, D-glucosamine, D-galactosamine, 1,2-diaminoethane, 1,2- or 1,3- diaminopropane, 1,3-diamino-2-propanol, 2-(2-aminoethylamino)ethylamine, 2-(2-aminoethylamino)ethanol, 3-(2-aminoethylamino)-propylamine and 3-(2-aminoethylamino)propanol.

**7.** Use according to Claim 5, **characterized in that** the compound containing an activated methylene functional group is chosen from 1,2,3,3-tetramethyl-3H-indolium iodide, 1,2,3,3-tetramethyl-3H-indolium p-toluenesulphonate, 1,2,3,3-tetramethyl-3H-indolium methanesulphonate, 1,3,3-trimethyl-2-methyleneindoline, 2,3-dimethylbenzothiazolium iodide, 2,3-dimethylbenzothiazolium p-toluenesulphonate, rhodanine, rhodanine acetic acid, 1-ethyl-2-quinaldinium iodide, 1-methyl-2-quinaldinium iodide, barbituric acid, thiobarbituric acid, 1,3-dimethylthiobarbituric acid, diethylthiobarbituric acid, oxindole, 3-indoxyl acetate, coumarone and 1-methyl-3-phenyl-2-pyrazinone.

**8.** Use according to any one of the preceding claims, **characterized in that** the composition has a pH of between 2 and 12, preferably between 3 and 11.

**9.** Use according to any one of the preceding claims, **characterized in that** the concentration of the polycylic aromatic vicinal triones of formula (I) or (II) is between 0.0001% and 30%, relative to the total weight of the composition.

**10.** Dyeing cosmetic composition containing at least one polycyclic aromatic vicinal trione of formula (I) or (II) as defined in Claim 1 and at least one surfactant and/or a polymeric agent of nonionic, cationic, anionic or amphoteric nature.

**11.** Ready-to-use dyeing cosmetic composition containing at least one polycyclic aromatic vicinal trione of formula (I) or (II) as defined in Claim 1 and at least one compound comprising a primary or secondary amine functional group or at least one compound comprising an activated methylene functional group or a mixture thereof.

**12.** Multicomponent dyeing agent containing

- as first component, a composition (a) containing at least one polycyclic aromatic vicinal trione of formula (I) or (II) as defined in Claim 1, and
- as second component, a composition (b) containing at least one activator as defined in any one of Claims 4 to 7.

**13.** Dyeing agent according to Claim 12, **characterized in that** it is provided in the form of a multicompartment kit, with at least a first compartment containing the composition (a) and at least a second compartment containing the composition (b).

**14.** Hair dyeing method comprising the application, to the hair, of a hair dyeing composition according to either of Claims 10 and 11, a sufficient leave-in time to allow the desired colour to be obtained, and then rinsing and washing the hair.

**15.** Hair dyeing method according to Claim 14, **characterized in that** it comprises heating hair impregnated with hair dyeing composition to a temperature of 80°C, preferably of 60°C.

**16.** Hair dyeing method comprising the application, to the hair, one after the other, in any order, of a composition (a) and a composition (b) as defined in Claim 12.

**17.** Hair dyeing method according to Claim 16, **characterized in that** an intermediate rinsing step is inserted between the application of the first composition and the application of the second composition.

**18.** Hair dyeing method according to either of Claims 16 and 17, comprising heating hair impregnated with the composition (a) and/or (b) to a temperature of 80°C, preferably of 60°C.

## Patentansprüche

**1.** Verwendung einer Zusammensetzung zur Färbung von Keratinsubstanzen, die in einem zum Färben geeigneten Medium, mindestens eine Verbindung der Formel (I) oder (II) enthält:

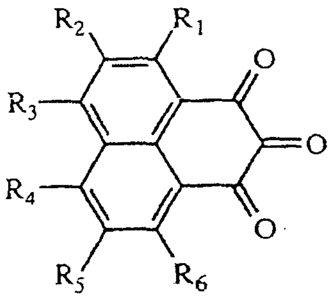

(I)

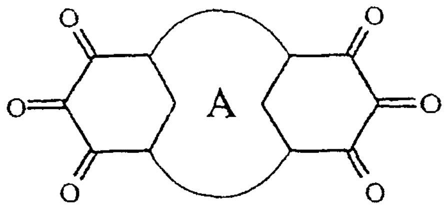

(II)

worin

$R^1$, $R^2$, $R^3$, $R^4$, $R^5$ und $R^6$ unabhängig voneinander jeweils ein Wasserstoffatom, ein Halogenatom, eine $C_1$-$_6$-Alkyl-, Hydroxy-, $C_{1-6}$-Alkoxy-, gegebenenfalls substituierte Benzoxy-, gegebenenfalls substituierte Aryloxy-, Amino-, Mono- oder Di-($C_{1-6}$-alkyl)-amino-, Mono- oder Di-($C_{1-6}$-hydroxyalkyl)-amino-, Thio-, $C_{1-6}$-Alkylthio-, $C_{1-6}$-Thioalkyl-, ($C_{1-6}$-Alkyl)-carbonyl-, Hydrogencarbonyl-, Hydroxycarbonyl-, ($C_{1-6}$-Alkoxy)-carbonyl-, Nitro-, Sulfonato- und Tri($C_{1-6}$-alkyl)-ammoniogruppe oder eine monocyclische oder polycyclische aromatische Gruppe mit mindestens 5 Ringgliedern mit kondensierten oder nicht kondensierten Ringen sind, die gegebenenfalls ein oder mehrere aus N, O, S und P ausgewählte Heteroatome enthält und gegebenenfalls einen oder mehrere Substituenten trägt, die aus Halogenatomen, $C_{1-6}$-Alkyl-, Hydroxy-, $C_{1-6}$-Alkoxy-, Amino-, Mono- oder Di-($C_{1-6}$-alkyl)-amino-, Mono- oder Di-($C_{1-6}$-hydroxyalkyl)-amino-, Thio-, $C_{1-6}$-Alkylthio-, $C_{1-6}$-Thioalkyl-, ($C_{1-6}$-Alkyl)-carbonyl-, Hydrogencarbonyl-, Hydroxycarbonyl-, ($C_{1-6}$-Alkoxy)-carbonyl-, Nitro-, Sulfonato- und Tri($C_{1-6}$-alkyl)-ammonio-, Phenoxy-, Imidazolyl- und Pyridinylgruppen ausgewählt sind, oder $R^1$ und $R^2$, $R^2$ und $R^3$, $R^3$ und $R^4$, $R^4$ und $R^5$ oder $R^5$ und $R^6$ zusammen jeweils zu zweit eine Gruppe -O-$CH_2$-O- oder einen aromatischen oder nicht aromatischen Ring mit 5 oder 6 Ringgliedern bilden, der gegebenenfalls ein oder mehrere aus N, O, S und P ausgewählte Heteroatome enthält und gegebenenfalls einen oder mehrere Substituenten trägt, die aus Halogenatomen, $C_{1-6}$-Alkyl-, Hydroxy-, $C_{1-6}$-Alkoxy-, Amino-, Mono- oder Di-($C_{1-6}$-alkyl)-amino-, Mono- oder Di-($C_{1-6}$-hydroxyalkyl)-amino-, Thio-, $C_{1-6}$-Alkylthio-, $C_{16}$-Thioalkyl-, ($C_{1-6}$-Alkyl)-carbonyl-, Hydrogencarbonyl-, Hydroxycarbonyl-, ($C_{1-6}$-Alkoxy)-carbonyl-, Nitro-, Sulfonato-, Tri($C_{1-6}$-alkyl)-ammonio-, Imidazolyl- und Pyridinylgruppen ausgewählt sind, und

A ein polycyclisches aromatisches System darstellt, das gegebenenfalls ein oder mehrere aus N, O, S und P ausgewählte Heteroatome enthält und gegebenenfalls einen oder mehrere Substituenten trägt, die aus den vorstehenden, durch $R^1$ bis $R^6$ dargestellten Substituenten der aromatischen Gruppen ausgewählt sind.

2. Verwendung nach Anspruch 1, **dadurch gekennzeichnet, dass** A einen Naphthalinkern darstellt, der durch Kondensation mit den beiden benachbarten $C_6$-Ringen ein Pyren-1,2,3,6,7,8-hexon der Formel (III) bildet

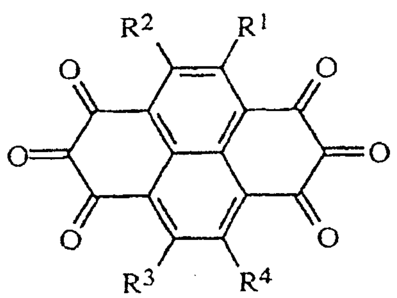

(III)

worin die Substituenten $R^1$ bis $R^4$ die im Anspruch 1 angegebene Bedeutung haben.

3. Verwendung nach Anspruch 1, **dadurch gekennzeichnet, dass** A einen Perylenkern darstellt, der durch Kondensation mit den beiden benachbarten $C_6$-Ringen ein Dibenzo-[cd,lm]-perylen-1,2,3,8,9,10-hexon der Formel (IV) bildet:

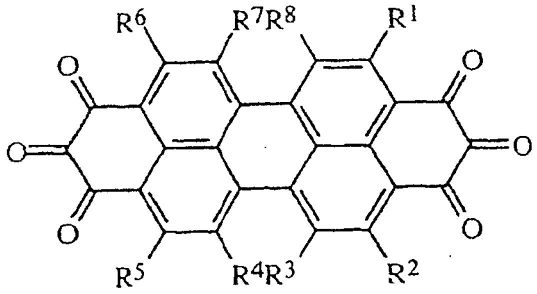

(IV)

worin die Substituenten $R^1$ bis $R^8$ die im Anspruch 1 für die Substituenten $R^1$ bis $R^6$ der Formel (I) angegebene Bedeutung haben.

4. Verwendung nach einem der Ansprüche 1 bis 3, **dadurch gekennzeichnet, dass** die Zusammensetzung außerdem mindestens einen Aktivator enthält, der es gestattet, die Kinetik der Reaktion der Ninhydrinverbindung mit der Keratinsubstanz zu modifizieren.

5. Verwendung nach Anspruch 4, **dadurch gekennzeichnet, dass** der Aktivator aus einem Oxidationsmittel, einem Reduktionsmittel, Brönsted-Säuren, einem Metallkatalysator, Proteinen, die Ionenstärke des Mediums modifizierenden Verbindungen, Verbindungen mit labilen Wasserstoffatomen, die aus denjenigen, die eine primäre oder sekundäre Aminfunktion tragen, und denjenigen, die eine aktivierte Methylenfunktion tragen, und aus deren Gemischen ausgewählt sind, ausgewählt ist.

6. Verwendung nach Anspruch 5, **dadurch gekennzeichnet, dass** die Verbindung, die eine primäre oder sekundäre Aminfunktion trägt, ein aromatisches Amin ist, das aus N,N-Dimethyl-p-phenylendiamin, N,N-Diethyl-p-phenylendiamin, N-(2-Hydroxyethyl)-N-ethyl-p-phenylendiamin, N,N-Bis-(2-hydroxyethyl)-p-phenylendiamin, N-(2-Methoxyethyl)-p-phenylendiamin, 2,3-, 2,4- oder 2,5-Dichlor-p-phenylendiamin, 2-Chlor-p-phenylendiamin, 2,5-Dihydroxy-4-morpholinoanilindibromhydrat, 2-, 3- oder 4-Aminophenol, 2-Aminomethyl-4-aminophenol, 2-Hydroxymethyl-4-aminophenol, ortho-Phenylendiamin, p-Phenylendiamin, ortho-Toluoldiamin, 2,5-Diaminotoluol, 2,5-Diaminophenol, 2,5-Diaminophenethol, 4-Amino-3-methylphenol, 2-(2,5-Diaminophenyl)-ethanol, 2,4-Diaminophenoxyethanol, 2-(2,5-Diaminophenoxy)-ethanol, 4-Methylaminoanilin, 3-Amino-4-(2'-hydroxyethyloxy)-anilin, 3,4-Methylendiaminoanilin, 3,4-Methylendioxyanilin, 3-Amino-2,4-dichlorphenol, 4-Methylaminophenol, 2-Methyl-5-aminophenol, 3-Methyl-4-aminophenol, 2-Methyl-5-(2-hydroxyethylamino)-phenol, 6-Methyl-3-amino-2-chlorphenol, 2-Methyl-5-amino-4-chlorphenol, 3,4-Methylendioxyphenol, 5-(2-Hydroxyethylamino)-4-methoxy-2-methylphenol, 4-Amino-2-hydroxymethylphenol, 1, 3-Diamino-2, '4-dimethoxybenzol, 2-, 3-, 4-Aminobenzoesäure, 2-Amino-, 3-Amino- oder 4-Aminophenylessigsäure, 2,3-, 2,4-, 2,5-, 3,4- oder 3,5-Diaminobenzoesäure, 4-Amino- oder 5-Aminosalicylsäure, 3-Amino-4-hydroxybenzoesäure, 4-Amino-3-hydroxybenzoesäure, 2-Amino-, 3-Amino- oder 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthonsäure, 3-Aminophthalsäure, 5-Aminoisophthalsäure, 1,3,5-Triaminobenzol, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminopyrokatechol, 4,6-Diaminopyrogallol, 3,5-Diamino-4-hydroxypyrokatechol und den aromatischen Anilinen und aromatischen Phenolen ausgewählt ist, die einen anderen aromatischen Rest tragen, entsprechend der Formel (II)

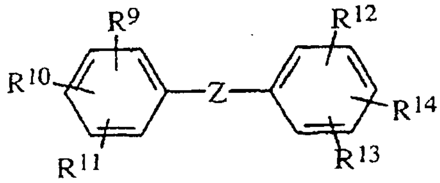

(II)

worin

$R_9$ eine Hydroxy- oder Aminogruppe darstellt, die gegebenenfalls mit einer $C_{1-4}$-Alkyl-, $C_{1-4}$-Hydroxyalkyl- oder ($C_{1-4}$-Alkoxy)-($C_{1-4}$-alkyl)-Gruppe substituiert sind,
$R^{10}$, $R^{11}$, $R^{12}$, $R^{13}$ und $R^{14}$ jeweils unabhängig ein Wasserstoffatom, eine Hydroxy- oder eine Aminogruppe darstellen, die gegebenenfalls mit einer $C_{1-4}$-Alkyl-, $C_{14}$-Hydroxyalkyl oder ($C_{1-4}$-Alkoxy)-($C_{1-4}$-alkyl)-Gruppe oder einer Carbon- oder Sulfonsäuregruppe substituiert sind,
Z eine direkte Bindung, eine gesättigte oder ungesättigte, gegebenenfalls hydroxylierte $C_{1-4}$-Kohlenwasserstoffkette, eine Carbonyl-, Sulfonyl- oder Iminogruppe, ein Sauerstoff- oder Schwefelatom oder eine Gruppe der Formel $Q-(CH_2-P-CH_2-Q')_o$ darstellt, worin P eine direkte Bindung oder eine Gruppe $-CH_2-$ oder -CHOH- darstellt, Q und Q' unabhängig voneinander ein Sauerstoffatom, eine Gruppe $NR^{15}$, worin $R^{15}$ ein Wasserstoffatom, eine $C_{1-4}$-Alkyl- oder $C_{1-4}$-Hydroxyalkylgruppe darstellt, oder eine Gruppe $0-(CH_2)_pNH$ oder $NH-(CH_2)_{p'}-O$ darstellen, worin p und p' den Wert 2 oder 3 haben, und o eine ganze Zahl zwischen 1 und 4 ist, oder ein aliphatisches Amin ist, das aus 2-Aminoethanol, 2-Methoxyethylamin, 2-Ethoxyethylamin, 2-(2-Aminoethoxy)-ethanol, 2- oder 3-Aminopropanol, 2,3-Dihydroxypropylamin, 4-Hydroxypropylamin, 2-Aminopropan-1,3-diol, 2-Amino-2-methylpropanol, 2-Amino-2-methylpropan-1,3-diol, 2-Amino-2-hydroxymethylpropan-1,3-diol, Tetrahydropentylamin, Pentahydroxyhexylaminen, wie Glucamin, D-Glucosamin, D-Galactosamin, 1,2-Diaminoethan, 1,2- oder 1,3-Diaminopropan, 1,3-Diamino-2-propanol, 2-(2-Aminoethylamino)-ethylamin, 2-(2-Aminoethylamino)-ethanol, 3-(2-Aminoethylamino)-propylamin und 3-(2-Aminoethylamino)-propanol ausgewählt ist.

**7.** Verwendung nach Anspruch 5, **dadurch gekennzeichnet, dass** die Verbindung mit aktivierter Methylenfunktion aus 1,2,3,3-Tetramethyl-3H-indoliumiodid, 1,2,3,3-Tetramethyl-3H-indolium-p-toluolsulfonat, 1,2,3,3-Tetramethyl-3H-indoliummethansulfonat, 1,3,3-Trimethyl-2-methylenindolin, 2,3-Dimethylbenzothiazoliumiodid, 2,3-Dimethylbenzothiazolium-p-toluolsulfonat, Rhodanin, Rhodaninessigsäure, 1-Ethyl-2-chinaldiniumiodid, 1-Methyl-2-chinaldiniumiodid, Barbitursäure, Thiobarbitursäure, 1,3-Dimethylthiobarbitursäure, Diethylthiobarbitursäure, Oxindol, 3-Indoxylacetat, Cumaron und 1-Methyl-3-phenyl-2-pyrazinon ausgewählt ist.

**8.** Verwendung nach einem der vorstehenden Ansprüche, **dadurch gekennzeichnet, dass** die Zusammensetzung einen pH zwischen 2 und 12, vorzugsweise zwischen 3 und 11, hat.

**9.** Verwendung nach einem der vorstehenden Ansprüche, **dadurch gekennzeichnet, dass** die Konzentration des oder der vicinalen aromatischen polycyclischen Trion(s/e) der Formel (I) oder (II) zwischen 0,0001% und 30%, bezogen auf das Gesamtgewicht der Zusammensetzung, beträgt.

**10.** Kosmetische Färbezusammensetzung, die mindestens ein vicinales aromatisches polycyclisches Trion der Formel (I) oder (II) nach Anspruch 1 und mindestens ein oberflächenaktives Mittel und/oder ein polymeres Mittel nichtionischer, kationischer, anionischer oder amphoterer Natur enthält.

**11.** Gebrauchsfertige kosmetische Färbezusammensetzung, die mindestens ein vicinales aromatisches polycyclisches Trion der Formel (I) oder (II) nach Anspruch 1 und mindestens eine Verbindung, die eine primäre oder sekundäre Aminfunktion enthält, oder mindestens eine Verbindung mit aktivierter Methylenfunktion oder ein Gemisch von diesen enthält.

**12.** Mehrkomponenten-Färbemittel, umfassend:

- als ersten Bestandteil eine Zusammensetzung (a), die mindestens ein vicinales aromatisches polycyclisches Trion der Formel (I) oder (II) nach Anspruch 1 enthält, und
- als zweiten Bestandteil eine Zusammensetzung (b), die mindestens einen Aktivator nach einem der Ansprüche 4 bis 7 enthält.

**13.** Färbemittel nach Anspruch 12, **dadurch gekennzeichnet, dass** es in Form eines Kits mit mehreren Unterteilungen mit mindestens einer ersten Unterteilung, die die Zusammensetzung (a) enthält, und mindestens einer zweiten Unterteilung, die die Zusammensetzung (b) enthält, vorliegt.

**14.** Verfahren zur Kapillarfärbung, umfassend das Aufbringen einer Kapillarfärbezusammensetzung nach einem der Ansprüche 10 und 11 auf die Haare, eine ausreichende Aufnahmezeit, um den Erhalt der gewünschten Färbung zu ermöglichen, und anschließend das Spülen und Waschen der Haare.

**15.** Verfahren zur Kapillarfärbung nach Anspruch 14, **dadurch gekennzeichnet, dass** es das Erwärmen der mit der Kapillarfärbezusammensetzung getränkten Haare bis auf eine Temperatur von 80°C, vorzugsweise 60°C, umfasst.

**16.** Verfahren zur Kapillarfärbung, umfassend das Aufbringen einer Zusammensetzung (a) und einer Zusammensetzung (b) nach Anspruch 12 nacheinander in einer beliebigen Reihenfolge auf die Haare.

**17.** Verfahren zur Kapillarfärbung nach Anspruch 16, **dadurch gekennzeichnet, dass** zwischen das Aufbringen der ersten Zusammensetzung und das Aufbringen der zweiten Zusammensetzung ein Spülschritt eingeschoben wird.

**18.** Verfahren zur Kapillarfärbung nach einem der Ansprüche 16 oder 17, umfassend das Erwärmen der mit der Zusammensetzung (a) und/oder (b) getränkten Haare bis auf eine Temperatur von 80°C, vorzugsweise 60°C.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- DE 4317855
- DE 19717222
- DE 19845481
- DE 19745355


### Littérature non-brevet citée dans la description


- **G. ERRERA.** *Gazz. Chim. Ital.,* 1913, vol. 431, 583
- **R. GLEITER ; R. KRAEMER ; H. IRNGARTINGER ; C. BISSINGER.** *J. Org. Chem.,* 1992, vol. 57, 252